# EUROPEAN PATENT APPLICATION

(11) **EP 3 045 200 A1**
(43) Date of publication of application: **20.07.2016**
(21) Application number: 15151369.4
(22) Date of filing: 16.01.2015
(51) Int. Cl.: A61M 25/04, A61J 1/10, A61M 5/148, A61J 11/04, B65D 75/58, A61M 5/46, B67B 7/00, A61M 5/162

(54) **Needle unit**

(71) Applicant: Sanofi-Aventis Deutschland GmbH, 65929 Frankfurt am Main (DE)
(72) Inventor: DITTRICH, Marcus-Meinolf, 65926 Frankfurt am Main (DE)
(74) Representative: Finger, Catrin

(57) **Abstract**

The invention relates to a needle unit (2) for connecting a drug delivery device to a medicament reservoir (1), the needle unit (2) comprising:
- a hollow cannula (3) having a sharp proximal tip (3.1) for piercing a reservoir wall (1.1) of the medicament reservoir (1),
- a stop member (5) which is axially fixable to the cannula (3),
- a sleeve (4) disposed on a section of the cannula (3), the sleeve (4) comprising a proximal section (4.1) and a folding section (4.3),
wherein in an initial state the folding section (4.3) is substantially concentrically arranged with respect to an outer surface of the cannula (3), wherein the folding section (4.3) is adapted to expand radially outwards by moving the proximal section (4.1) towards the stop member (5).

## Description

### Technical Field

The invention relates to a needle unit for connecting a drug delivery device to a medicament reservoir.

### Background of the Invention

A Drug delivery device may apply medicament reservoirs which need to be kept sterile prior to use and which may be connected to the drug delivery device by piercing a reservoir wall of the reservoir with a hollow needle. In particular with a flexible reservoir wall, it may be difficult to establish and maintain the connection between the needle and the reservoir.

Thus, there remains a need for an improved needle unit.

### Summary of the Invention

It is an object of the present invention to provide an improved needle unit.

The object is achieved by a needle unit according to claim 1.

Exemplary embodiments of the invention are given in the dependent claims.

According to the invention, a needle unit for connecting a drug delivery device to a medicament reservoir comprises:
- a hollow cannula having a sharp proximal tip for piercing a reservoir wall of the medicament reservoir,
- a stop member which is axially fixable to the cannula,
- a sleeve disposed on a section of the cannula, the sleeve comprising a proximal section and a folding section,
   wherein in an initial state, the folding section is substantially concentrically arranged with respect to an outer surface of the cannula, wherein the folding section is adapted to expand radially outwards by moving the proximal section towards the stop member. Thus, the position of the cannula within the reservoir is defined and can be reliably maintained.

In an exemplary embodiment, the folding section is arranged between the proximal section and the stop member.

In an exemplary embodiment, the folding section comprises a number of stretchers separated by slots. If the proximal section is moved towards the stop member, the stretchers of the folding section open up similar to an umbrella or an expansion anchor.

In an exemplary embodiment, the stretchers respectively comprise at least one pivot or weakened area defining a bending or folding behaviour of the folding section.

In an exemplary embodiment, the folding section has a reduced wall thickness compared to the proximal section. The folding section is thus arranged to expand radially outwards when being compressed axially in a similar way as a blind rivet or an expansion anchor. A wall thickness and material of the folding section may be selected to subject the folding section only to elastic deformation thus rendering the needle unit re-usable.

In an exemplary embodiment, the cannula near the proximal tip comprises an external thread adapted to threadedly engage an internal thread within the proximal section of the sleeve. The folding section may thus be expanded by rotating the needle relative to the sleeve, e. g. after having pierced a medicament wall with the cannula.

In an exemplary embodiment, the sleeve is coupled to the stop member in a manner preventing relative rotation, thus facilitating relative rotation between the cannula and the sleeve once the needle unit is inserted into a medicament container.

In an exemplary embodiment, the sleeve comprises or consists of steel, e. g. the same steel as the cannula.

In an exemplary embodiment, the sleeve comprises or consists of a plastic material, e.g. a bio compatible and/or drug compatible material which does not react with the medicament held in the medicament reservoir. In particular, the material may be polytetrafluoroethylene or polyurethane.

In an exemplary embodiment, a tapering section is arranged at the proximal tip or on the proximal section, the tapering section providing a transition between an external diameter of the proximal tip and an external diameter of the proximal section. Insertion of the needle unit into a medicament reservoir is thus facilitated while the risk of damaging the reservoir wall of the medicament reservoir is mitigated.

In an exemplary embodiment, the stop member is arranged as a gap ring sealing the stop member against the cannula.

In an exemplary embodiment, the stop member comprises or consists of a resilient rubber material or latex in order to be capable of forming a sealing surface against the reservoir wall and capable of forming a mechanical stop for limiting the extent of the cannula penetrating into the medicament reservoir.

In an exemplary embodiment, the stop member is arranged as a second folding section of the sleeve arranged distally from the folding section, so the reservoir wall may be squeezed between the two folding sections. The second folding section may be of the type having the stretchers or of the type having the reduced wall thickness. The two folding sections may be of the same type or of different types.

In an exemplary embodiment, the sleeve comprises a distal section having internal threads adapted to engage respective external threads on the cannula. The second folding section may thus expand by rotating the cannula relative to the distal section.

In an exemplary embodiment, the internal thread in the proximal section and the corresponding external thread on the cannula have the opposite handedness of the internal thread in the distal section and the corresponding external thread on the cannula. For example, one of the threads may be right-handed and the other left-handed. Both folding sections may thus be expanded by rotating the needle within the sleeve. The folding sections may be connected to each other such that the sleeve rotates as a whole relative to the cannula.

In an exemplary embodiment, the reservoir wall is flexible.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic view of an exemplary embodiment of a needle unit prior to use,
- Figure 2: is a schematic view of the needle unit of figure 1 inserted into a medicament reservoir,
- Figure 3: is a schematic view of another exemplary embodiment of a needle unit prior to use,
- Figure 4: is a schematic view of the needle unit of figure 3 inserted into a medicament reservoir,
- Figure 5: is a schematic detail view of figure 4,
- Figure 6: is a schematic view of yet another exemplary embodiment of a needle unit, and
- Figure 7: is a schematic view of the needle unit inserted into a medicament reservoir.

Corresponding parts are marked with the same reference symbols in all figures.

### Detailed Description

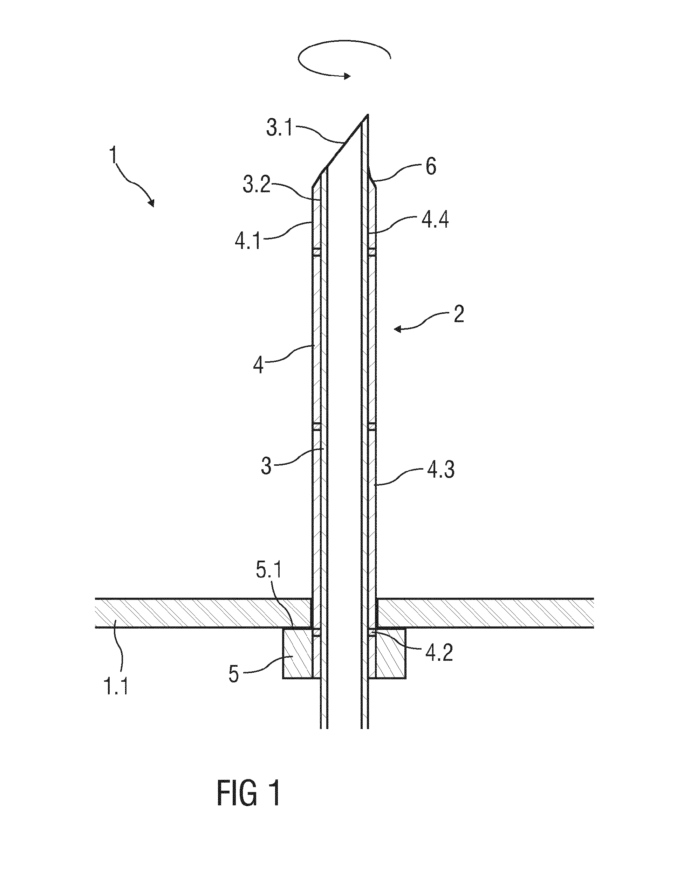

Figure 1 is a schematic view of a medicament reservoir 1 with an exemplary embodiment of a needle unit 2. In an exemplary embodiment, the medicament reservoir 1 may have a flexible reservoir wall 1.1. The needle unit 2 comprises a hollow needle or cannula 3 for establishing a fluid connection between the medicament reservoir 1 and a drug delivery device (not shown). The longitudinal extension of the cannula 3 defines an axial direction. The cannula 3 comprises a sharp proximal tip 3.1 intended to pierce the reservoir wall 1.1 of the medicament reservoir 1. The drug delivery device may comprise a medicament pump (not shown) for pumping a medicament from the medicament reservoir 1 through the cannula 3. Furthermore, the needle unit 2 comprises a sleeve 4 disposed on a section of the cannula 3. In an initial state as shown in figure 1, the fluid sleeve 4 is substantially concentrically arranged with respect to the cannula 3.

The needle unit 2 further comprises a stop member 5 which is axially fixed to the cannula 3 but rotatable relative to the cannula 3. The purpose of the stop member 5 is to limit movement of the needle unit 2 towards the medicament reservoir 1 when being attached to it. The stop member 5 may have a cylindrical form or any other form. In an exemplary embodiment, a surface 5.1 of the stop member 5 facing the proximal tip 3.1 is shaped to allow for sealing the needle unit 2 against an outer surface of the reservoir wall 1.1 when the stop 5 is pushed against the reservoir wall 1.1.

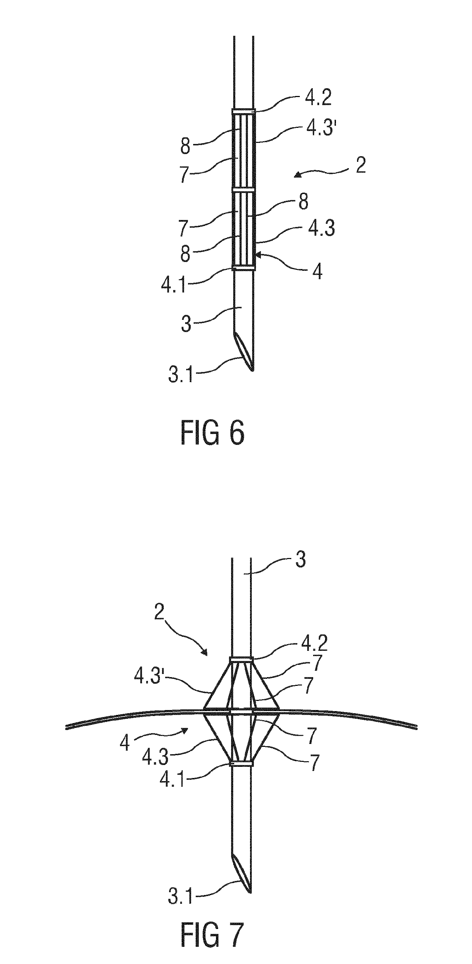

In an exemplary embodiment, the sleeve 4 comprises a ring shaped proximal section 4.1 and a ring shaped distal section 4.2 adjacent the stop member 5 and a folding section 4.3 between the proximal section 4.1 and the distal section 4.2, wherein the folding section 4.3 comprises a number of longitudinal stretchers separated by longitudinal slots (not illustrated in figure 1, cf. fig. 6). The stretchers may comprise a pivot or a weakened area, e. g. halfway or at another point along its length, defining a bending or folding behaviour of the folding section 4.3 when the proximal section 4.1 and distal section 4.2 are moved towards each other. Similar pivots or weakened areas may be provided for connecting the stretchers to the proximal section 4.1 and/or the distal section 4.2. The sleeve 4 is coupled to the stop member 5 in a manner preventing relative rotation, e.g. splined or glued. In an exemplary alternative embodiment, the stretchers of the folding section 4.3 are attached to or guided into the stop member 5 so the sleeve 4 does not have a distal section.

In an exemplary embodiment, the sleeve 4 comprises or consists of steel, e.g. the same steel as the cannula 3.

The cannula 3 may comprise a tapering section 6 at the proximal tip 3.1, the tapering section 6 adapted to smoothen a transition between an external diameter of the proximal tip 3.1 of the cannula 3 and an external diameter of the sleeve 4, i.e. the proximal section 4.1, which is slightly greater than that of the proximal tip 3.1. In an alternative embodiment the tapering section 6 may be arranged on the sleeve 4.

The cannula 3 near the proximal tip 3.1 comprises an external thread 3.2 adapted to threadedly engage an internal thread 4.4 within the proximal section 4.1 of the sleeve 4.

The stop member 5 may be arranged as a gap ring preventing liquid from flowing between the stop member 5 and the cannula 3.

In figure 1, the needle unit 2 is partially deployed, i.e. the cannula 3 is inserted in the medicament reservoir 1 through the reservoir wall 1.1. The sharp proximal tip 3.1 of the cannula 3 pierces the reservoir wall 1.1 when pushed against it. The cannula 3 is inserted into the medicament reservoir 1 to the point where the stop member 5 abuts the outer surface of the reservoir wall 1.1. Subsequently, the cannula 3 is rotated relative to the stop member 5 and hence relative to the sleeve 4. Due to the threaded engagement between the proximal section 4.1 of the sleeve 4 and the cannula 3, the proximal section 4.1 moves towards the stop member 5 thereby opening up and deploying the folding section 4.3 similar to an umbrella or an expansion anchor.

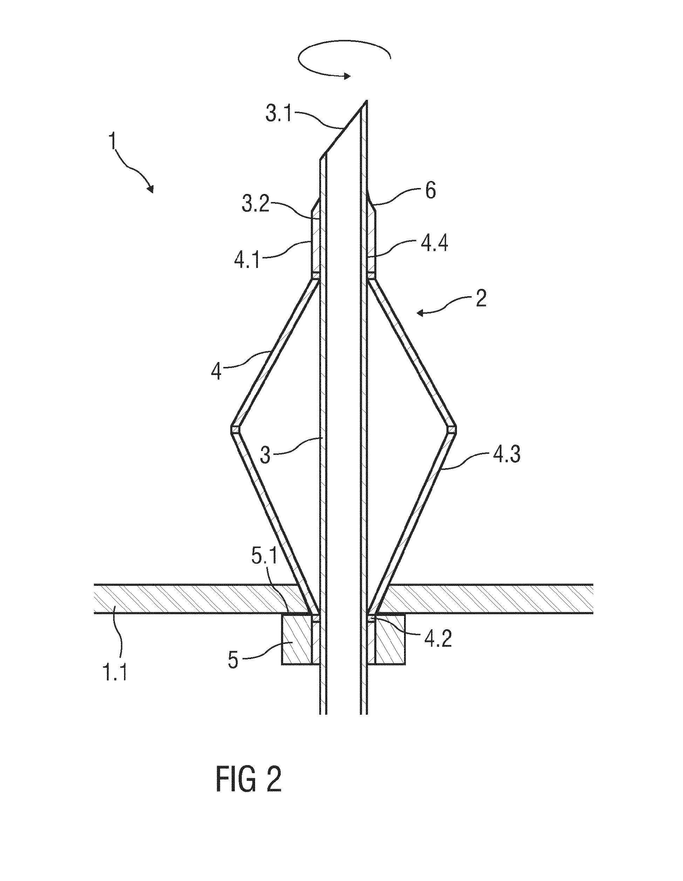

Figure 2 is a schematic view of the medicament reservoir 1 with the needle unit 2, wherein the folding section 4.3 is opened up and deployed to a point where it forms a second stop inside the medicament reservoir 1 for limiting movement of the needle unit 2 in the axial direction out of the medicament reservoir 1. The reservoir wall 1.1 is thus squeezed between the stop member 5 and the folding section 4.3.

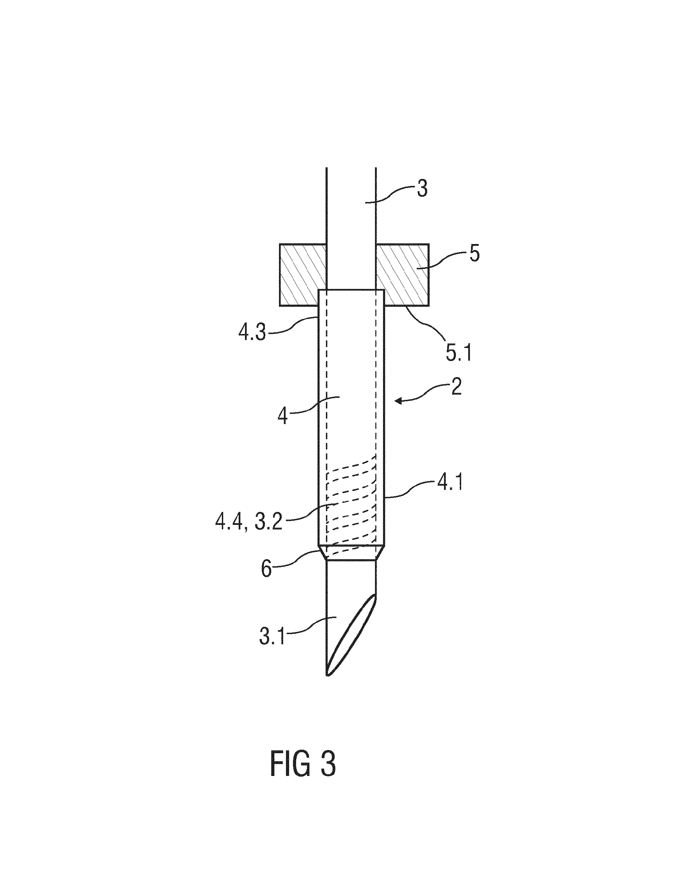

Figure 3 is a schematic view of another exemplary embodiment of a needle unit 2 prior to use. The needle unit 2 comprises a hollow needle or cannula 3 for establishing a fluid connection between a medicament reservoir and a drug delivery device (not shown). The longitudinal extension of the cannula 3 defines an axial direction. The cannula 3 comprises a sharp proximal tip 3.1 intended to pierce a reservoir wall of a medicament reservoir. The drug delivery device may comprise a medicament pump (not shown) for pumping a medicament from the medicament reservoir through the cannula 3. Furthermore, the needle unit 2 comprises a sleeve 4 disposed on a section of the cannula 3. In an initial state as shown in figure 3, the sleeve 4 is substantially concentrically arranged with respect to the cannula 3.

The needle unit 2 further comprises a stop member 5 which is axially fixed to the cannula 3 but rotatable relative to the cannula 3. The purpose of the stop member 5 is to limit movement of the needle unit 2 towards the medicament reservoir when being attached to it. The stop member 5 may have a cylindrical form or any other form. In an exemplary embodiment, a surface 5.1 of the stop member 5 facing the proximal tip 3.1 is shaped to allow for sealing the needle unit 2 against an outer surface of the reservoir wall when the stop 5 is pushed against the reservoir wall.

In an exemplary embodiment, the sleeve 4 comprises a proximal section 4.1 having an internal thread 4.4 adapted to engage an external thread 3.2 of the cannula 3. The sleeve 4 furthermore comprises a folding section 4.3 adjacent the stop member 5, the folding section 4.3 arranged to expand radially outwards when being compressed axially in a similar way as a blind rivet. This may be achieved by the folding section 4.3 having a reduced wall thickness compared to the proximal section 4.1. The sleeve 4 is coupled to the stop member 5 in a manner preventing relative rotation, e.g. by splining or gluing.

In an exemplary embodiment, the sleeve 4 comprises or consists of a plastic material. In an exemplary embodiment, the sleeve 4 comprises or consists of a bio compatible and/or drug compatible material which does not react with the medicament held in the medicament reservoir 1. For example, the sleeve 4 may be made from the same material as that used for peripheral venous catheters. For example, this material may be polytetrafluoroethylene or polyurethane.

The sleeve 4 may comprise a tapering section 6 at the proximal section 4.1, the tapering section 6 adapted to smoothen a transition between an external diameter of the proximal tip 3.1 of the cannula 3 and an external diameter of the proximal section 4.1, which is slightly greater than that of the proximal tip 3.1. In an alternative embodiment, the tapering section 6 may be arranged on the cannula 3.

The stop member 5 may be arranged as a gap ring preventing liquid from flowing between the stop member 5 and the cannula 3.

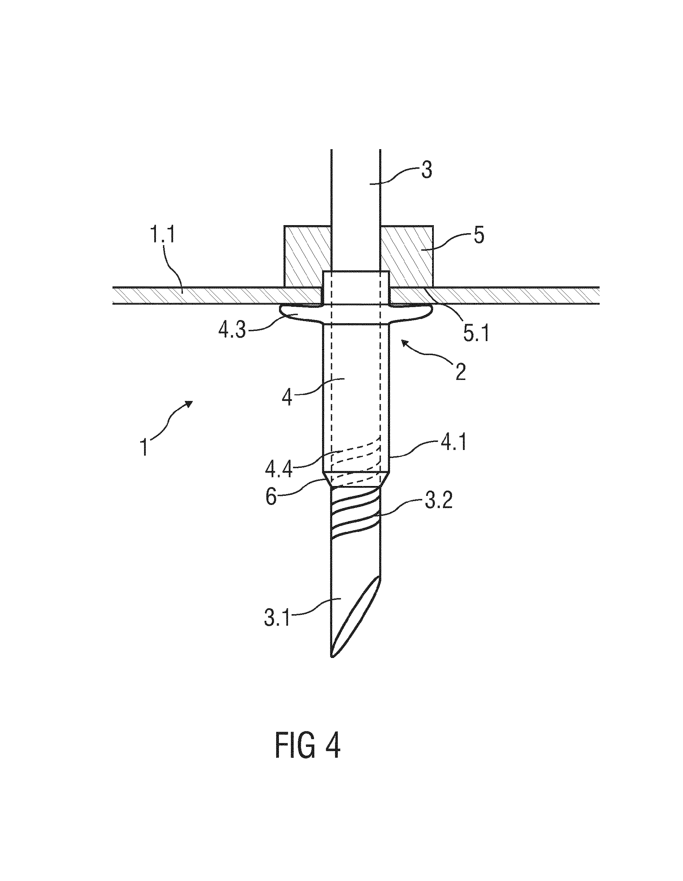

Figure 4 is a schematic view of a medicament reservoir 1 with the needle unit 2, wherein the cannula 3 is inserted in the medicament reservoir 1 through the reservoir wall 1.1. The sharp proximal tip 3.1 of the cannula 3 pierces the reservoir wall 1.1 when pushed against it. The cannula 3 is inserted into the medicament reservoir 1 to the point where the stop member 5 abuts the outer surface of the reservoir wall 1.1. Subsequently, the cannula 3 is rotated relative to the stop member 5 and hence relative to the sleeve 4. Due to the threaded engagement between the proximal section 4.1 of the sleeve 4 and the cannula 3, the proximal section 4.1 moves towards the stop member 5 thereby opening up and deploying the folding section 4.3 to a point where it forms a second stop inside the medicament reservoir 1 for limiting movement of the needle unit 2 in the axial direction out of the medicament reservoir 1.

The reservoir wall 1.1 is thus squeezed between the stop member 5 and the folding section 4.3.

A wall thickness and material of the folding section 4.3 may be selected to subject the folding section 4.3 only to elastic deformation, thus rendering the needle unit 2 re-usable. In this case, rotation between the sleeve 4 and the cannula 3 may be reversible so that the sleeve 4 can be returned into the initial flush configuration illustrated in figure 1 or figure 3.

In an exemplary embodiment, sealing of the needle unit 2 within the reservoir wall 1.1 is achieved by the reservoir wall 1.1 being sufficiently compliant to form a sealing line between an outer surface of the cannula 3 and a circumference of a hole cut into the reservoir wall 1.1 upon penetration of the cannula 3. Furthermore, a pressure difference may exist between the reservoir 1 and the environment such that the pressure within the medicament reservoir 1 is lower than in the environment due to suction of a pump of a drug delivery device. This pressure difference may also contribute to avoiding leakage.

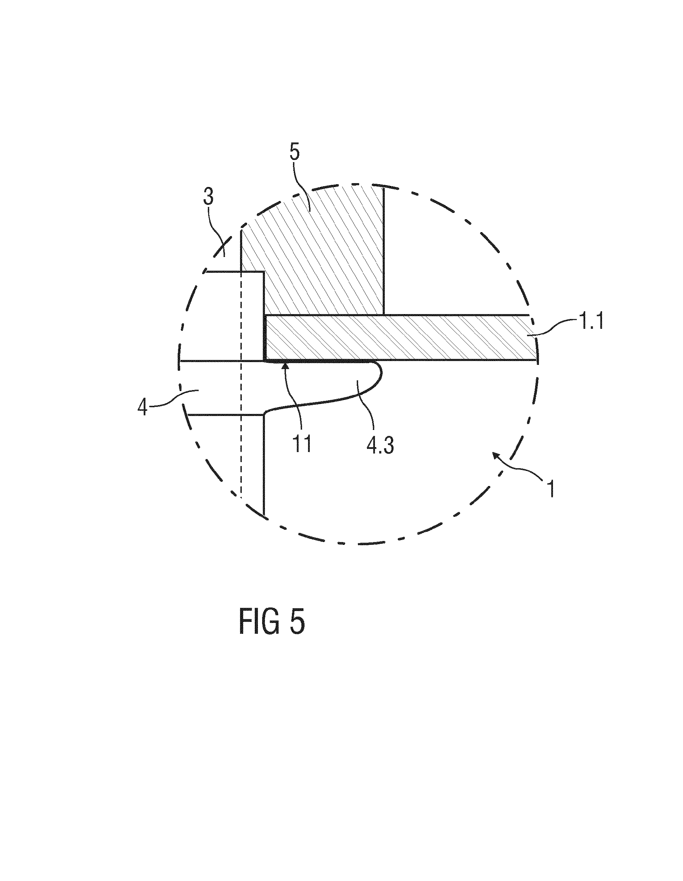

Figure 5 is a schematic detail view of the needle unit 2 and the medicament reservoir 1 of figure 4 with the expanded folding section 4.3. It can be seen that the expanded folding section 4.3 forms a sealing area 11 to ensure that the medicament inside the medicament reservoir 1 leaves the medicament reservoir 1 only through the hollow cannula 3 and does not leak out of the medicament reservoir 1 outside of the cannula 3. The reservoir wall 1.1 is squeezed between the folding section 4.3 and the surface 5.1 of the stop member 5 such that liquid cannot escape from the medicament reservoir 1 unless being drawn through the lumen within the cannula 3.

Figure 6 is a schematic view of yet another exemplary embodiment of a needle unit 2. Figure 7 shows the needle unit 2 inserted into a medicament reservoir 1. This embodiment is similar to the one of figures 1 and 2 but comprises a stop member 5 in the shape of a second folding section 4.3', so the reservoir wall 1.1 is squeezed between the two folding sections 4.3, 4.3'.

In an exemplary embodiment, the sleeve 4 comprises a ring shaped proximal section 4.1 and a ring shaped distal section 4.2, wherein the folding section 4.3 is arranged adjacent the proximal section 4.1 and the second folding section 4.3' is arranged adjacent the distal section 4.2. Both folding sections 4.3, 4.3' respectively comprise a number of stretchers 7 separated by longitudinal slots 8. The stretchers 7 may comprise a pivot or a weakened area, e. g. halfway or at another point along its length, defining a bending or folding behaviour of the folding section 4.3, 4.3' when the proximal section 4.1 and distal section 4.2 are moved towards each other. Similar pivots or weakened areas may be provided for connecting the stretchers 7 to the proximal section 4.1 and/or the distal section 4.2. Both the proximal section 4.1 and the distal section 4.2 have internal threads 4.4 adapted to engage respective external threads 3.2 on the cannula 3, wherein the internal thread 4.4 in the proximal section 4.1 and the corresponding external thread 3.2 on the cannula 3 has the opposite handedness of the internal thread 4.4 in the distal section 4.2 and the corresponding external thread 3.2 on the cannula 3.

In an exemplary embodiment, the cannula 3 comprises or consists of steel.

The stop member 5 may comprise or consist of a resilient rubber material or latex that is capable of forming a sealing surface and capable of forming a mechanical stop for limiting the extent of the cannula 3 penetrating into the medicament reservoir 1.

The needle unit 2 may be reusable.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: medicament reservoir
- 1.1: reservoir wall
- 2: needle unit
- 3: cannula
- 3.1: proximal tip
- 3.2: external thread
- 4: sleeve
- 4.1: proximal section
- 4.2: distal section
- 4.3: folding section
- 4.4: internal thread
- 5: stop member
- 5.1: surface
- 6: tapering section
- 7: stretcher
- 8: slot
- 11: sealing area

## Claims

1. Needle unit (2) for connecting a drug delivery device to a medicament reservoir (1), the needle unit (2) comprising:
- a hollow cannula (3) having a sharp proximal tip (3.1) for piercing a reservoir wall (1.1) of the medicament reservoir (1),
- a stop member (5) which is axially fixable to the cannula (3),
- a sleeve (4) disposed on a section of the cannula (3), the sleeve (4) comprising a proximal section (4.1) and a folding section (4.3),
wherein in an initial state, the folding section (4.3) is substantially concentrically arranged with respect to an outer surface of the cannula (3), wherein the folding section (4.3) is adapted to expand radially outwards by moving the proximal section (4.1) towards the stop member (5).

2. Needle unit (2) according to claim 1, wherein the folding section (4.3) is arranged between the proximal section (4.1) and the stop member (5).

3. Needle unit (2) according to any one of the claims 1 and 2, wherein the folding section (4.3) comprises a number of stretchers (7) separated by slots (8).

4. Needle unit (2) according to claim 3, wherein the stretchers (7) respectively comprise at least one pivot or weakened area.

5. Needle unit (2) according to any one of the claims 1 and 2, wherein the folding section (4.3) has a reduced wall thickness compared to the proximal section (4.1).

6. Needle unit (2) according to any one of the preceding claims, wherein the sleeve (4) is coupled to the stop member (5) in a manner preventing relative rotation.

7. Needle unit (2) according to any one of the preceding claims, wherein the sleeve (4) comprises or consists of steel.

8. Needle unit (2) according to any one of the claims 1 to 6, wherein the sleeve (4) comprises or consists of a plastic material, in particular polytetrafluoroethylene or polyurethane.

9. Needle unit (2) according to any one of the preceding claims, wherein a tapering section (6) is arranged at the proximal tip (3.1) or on the proximal section (4.1), the tapering section (6) providing a transition between an external diameter of the proximal tip (3.1) and an external diameter the proximal section (4.1).

10. Needle unit (2) according to any one of the preceding claims, wherein the cannula (3) near the proximal tip (3.1) comprises an external thread (3.2) adapted to threadedly engage an internal thread (4.4) within the proximal section (4.1) of the sleeve (4).

11. Needle unit (2) according to any one of the preceding claims, wherein the stop member (5) is arranged as a gap ring sealing the stop member (5) against the cannula (3).

12. Needle unit (2) according to any one of the preceding claims, wherein the stop member (5) comprises or consist of a resilient rubber material or latex.

13. Needle unit (2) according to any one of the claims 1 to 11, wherein the stop member (5) is arranged as a second folding section (4.3') of the sleeve (4) arranged distally from the folding section (4.3).

14. Needle unit (2) according to claim 13, wherein the sleeve (4) comprises a distal section (4.2) having internal threads (4.4) adapted to engage respective external threads (3.2) on the cannula (3).

15. Needle unit (2) according to claim 14, wherein the internal thread (4.4) in the proximal section (4.1) and the corresponding external thread (3.2) on the cannula (3) has the opposite handedness of the internal thread (4.4) in the distal section (4.2) and the corresponding external thread (3.2) on the cannula (3).
